# EUROPEAN PATENT APPLICATION

(11) **EP 1 989 991 A1**
(43) Date of publication of application: **12.11.2008**
(21) Application number: 07425263.6
(22) Date of filing: 07.05.2007
(51) Int. Cl.: A61B 1/00

(54) **Protective device for endoscopic apparatus**

(71) Applicant: Endoclarix S.r.L., 41037 Mirandola (Modena) (IT)
(72) Inventor: Greco, Francesco, 22063 Cantu' (Como) (IT)
(74) Representative: Lunati, Vittoriano

(57) **Abstract**

There is disclosed a protective device (1) for endoscopic apparatus (10), including an elongated and flexible probe (11) and comprising a lens (13) at the distal end, the device (1) comprising: a window (2), suitable to cover the lens (13), a connection element (3), suitable to constrain the protective device (1) to the endoscopic apparatus (10), a flexible tubular sheath (4) suitable to cover at least part of the probe (11), and connected at the ends to the window (2) and to the connection element (3) and comprising a first portion (4a), extending from the connection element (3) for a length of between 3 cm and 20 cm and a second portion (4b) complementary with respect to the first portion (4a), and in that said first portion (4a) has a greater flexural rigidity with respect to said second portion.

## Description

The present invention relates to a protective device for endoscopic apparatus and a process for the realization of this device of the type specified in the preamble of the independent claims.

There are currently known different endoscopic devices used in the medical field.

They allow internal organs of the human body to be viewed by means of micro cameras which can be inserted therein.

The use of endoscopic apparatus in medicine has the important advantage of allowing observation of the internal organs of the human body without having to make cuts or incisions or by making simple keyhole incisions rather than large openings.

In particular, there have been developed oesophageal endoscopic apparatus, to analyze the digestive tract, and endoscopic apparatus that allow the heart, arteries, intestine, respiratory tract and substantially all organs of the human body to be viewed.

These endoscopic apparatus are essentially realized by a probe, suitable to be inserted inside the human body, and by control means suitable to allow control of the apparatus and viewing of the images.

In particular, the probe comprises a camera lens positioned at the distal end of a flexible tube, suitable to functionally and mechanically connect the lens to the control means.

It is indispensable that endoscopic apparatus, and in particular probes, which come into contact with the inside of the human body, do not act as means for spreading bacteria and impurities and therefore they must be sterilized before use.

However, sterilization of endoscopic apparatus has some important drawbacks.

Firstly, sterilization of these apparatus causes considerable loss of time and consequently the apparatus remains out of use for lengthy periods of time.

This fact is particularly negative due to the fact that endoscopic apparatus are very costly and that for a hospital, or medical practice, it would be too expensive to purchase a plurality of endoscopic apparatus to use alternatively to and synchronously with the sterilization processes of some of these apparatus.

Moreover, these endoscopic apparatus comprise very fragile portions and elements which can be damaged during sterilization.

Consequently, endoscopic apparatus are not sterilized, but only disinfected by means of specific liquid disinfectants at low pressure and temperatures. Nonetheless, the disinfectant liquids are by nature toxic for the human body and must therefore be carefully rinsed, which prolongs the disinfection period.

To overcome the aforesaid drawbacks, there have been realized disposable protective sheaths for these apparatus and in particular for the probes.

These protective sheaths are substantially realized by a transparent film made of flexible elastic material which can be applied around the probe and which is preferably fastened to the control means.

These sheaths also have some important drawbacks.

In fact, they are preferably realized to adhere tightly to the probes, so as not to cause a visual impediment for the lens located therein and, due to said adhesion, it is difficult to remove the sheath from the flexible probe, which during removal twists around itself.

This drawback can be partly overcome by the addition of a lubricating agent, such as talc, interposed between the probes and the sheaths.

Nonetheless, said lubricating agent can cause a visual impediment for the lens and in any case is a substance that can cause damage inside the human body.

Alternatively, the sheaths can be cut, but with this process there is the risk of cutting and damaging the probe.

In this situation the technical aim underlying the present invention is to devise a protective device for endoscopic apparatus capable of substantially overcoming the aforesaid drawbacks.

Within the scope of said technical aim, an important object of the invention is to devise a protective device for endoscopic apparatus suitable to allow easy removal of the device.

A further object of the invention is to obtain a protective device for endoscopic apparatus without the use of foreign bodies that can be released outside said device.

Another important object of the invention is to obtain a protective device for endoscopic apparatus which does not obstruct the visibility and manageability thereof.

Last but not least object of the invention is to realize an inexpensive protective device for endoscopic apparatus.

The technical aim and objects specified are achieved by a protective device for endoscopic apparatus according to the appended Claim 1.

Preferred embodiments are highlighted in the dependent claims.

The characteristics and advantages of the invention are better specified below by the detailed description of a preferred embodiment of the invention, with reference to the accompanying drawings, wherein:

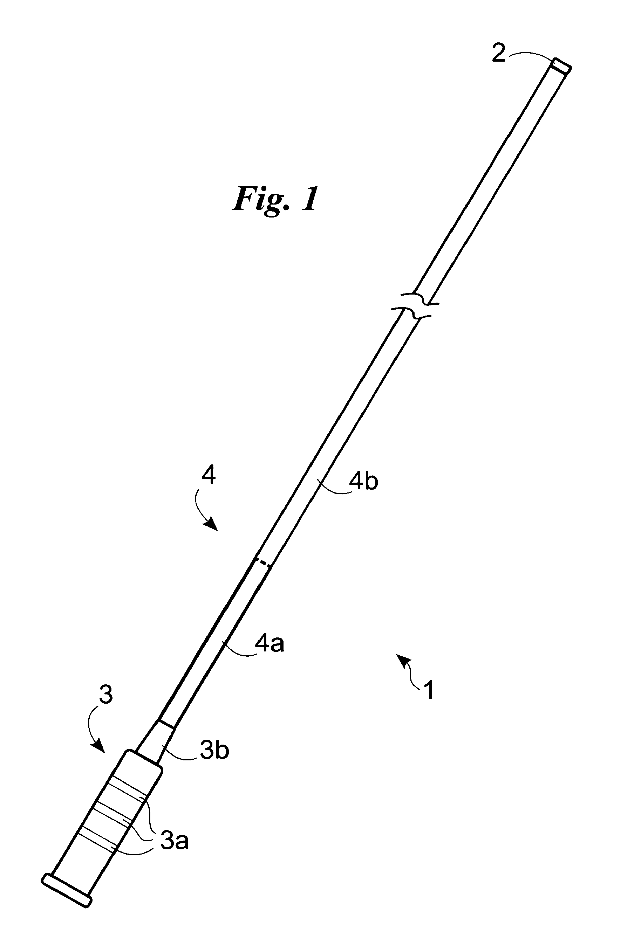

- **Fig. 1** shows the device according to the invention;

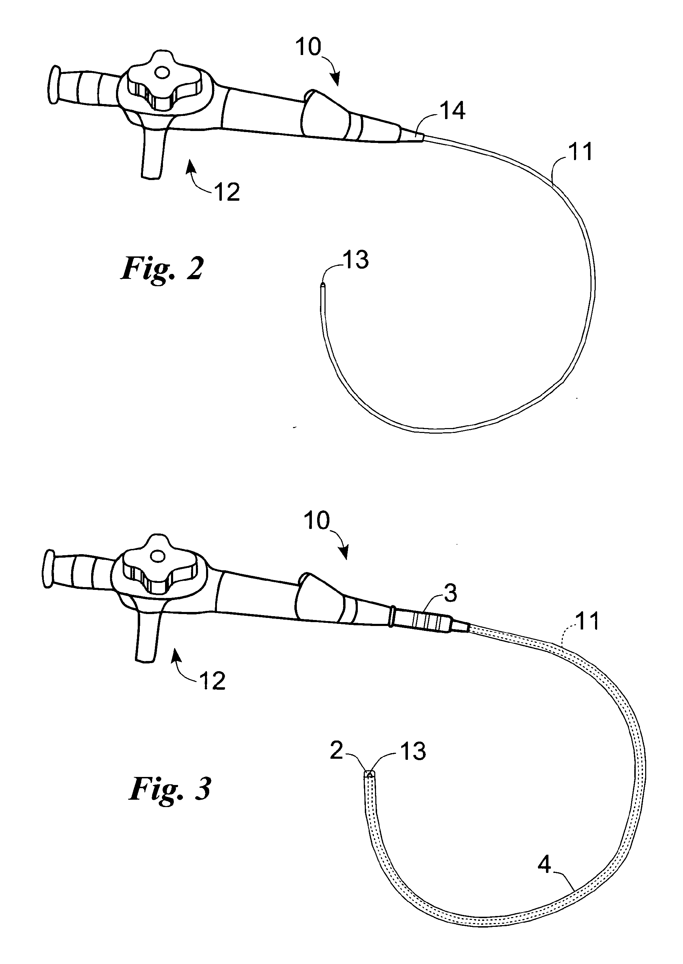

- **Fig. 2** shows an endoscopic apparatus without the device according to the invention; and
- **Fig. 3** shows an endoscopic apparatus on which the device according to the invention is positioned.

With reference to the aforesaid Figures, the protective device according to the invention is indicated as a whole with the number **1.**

It is suitable to be applied to an endoscopic apparatus **10,** and in particular to an oesophageal endoscope or to other known types of endoscopes.

The endoscopic apparatus 10 comprises an elongated and flexible probe **11** and control means **12** of the apparatus 10.

More specifically, the probe 11 is suitable to be inserted inside the human body and comprises at the distal end a lens **13** of a camera or the like, suitable to film or photograph the inside of the human body.

The probe 11 is connected to the control means 12 at the proximal end by means of a coupling element **14.**

It is realized by a flexible tube preferably coated with a covering made of polymeric material and including mechanical and functional connection means between the lens 13 and the control means 12.

This connection means is realized in particular by optical fibres or by electrical connections or the like.

The control means 12 of the apparatus 10 is suitable to allow control of the probe 11 by an operator, and in particular a physician, and also transfer of the images to a specific viewing means, for example realized by a monitor.

The protective device 1 comprises, briefly, a window **2,** suitable to cover the lens 13, a connection element **3,** suitable to constrain the protective device 1 to the endoscopic apparatus 10, and a tubular sheath **4** suitable to cover at least part of the probe 11 and abutting at the proximal end with the connection element 3 and at the distal end with the window 2. ln particular, the tubular sheath 4 is a flexible sheath and preferably elastic, made of polymeric material and preferably of polyvinyl chloride.

It has a diameter proportional to, and slightly larger than, the diameter of the probe 11. More specifically, if applied to an oesophageal endoscope, it has a diameter of between 2 mm and 6 mm and preferably of around 4 mm.

The tubular sheath 4 also has a length proportional to the length of the probe 11, and in particular a length slightly less than the length of the probe 11. lf applied to an oesophageal endoscope, the sheath 4 has a length of between 10 cm and 40 cm and in particular of around 23 cm.

The tubular sheath 4 has walls with a thickness of less than one millimetre and in particular between 0.2 mm and 0.3 mm.

It also comprises a first portion **4a,** extending from the connection element 3 and a second portion **4b** complementary with respect to said first portion and abutting with the window 2.

Advantageously the first portion 4a has a greater flexural rigidity with respect to the second portion 4b, in particular a flexural rigidity of over 30% and preferably of 50% with respect to the flexural rigidity of the second portion 4b.

In particular if the tubular sheath 4, including the window 2, and having a length of around 23 cm, a diameter of around 4 mm, a wall with a thickness of around 0.2-0.3 mm and a mass of around 0.6-0.7 g, is constrained by the proximal end, and is subjected to its own load, it has a bending deflection, at the distal end, of between 5 and 10 cm and in particular of around 7 cm, while the first portion 4a, having a length of around 5-7 cm, has a maximum deflection of less than 1 cm.

In order to obtain increased rigidity, the first portion 4a can have a wall of greater thickness than the second portion 4b, be realized with a different material or be subjected to a deplasticizing process, as described hereunder.

The first portion 4a has a length of between 3 cm and 20 cm, preferably between 5 cm and 15 cm and more preferably of around 6-7 cm.

Moreover, this length corresponds to a portion of between one half and one tenth of the length of the entire tubular sheath 4, and preferably of around one third of the length of the entire tubular sheath 4.

As specified previously, the tubular sheath 4 abuts distally with the window 2 and proximally with the connection element 3.

The window 2 is suitable to cover the lens 13.

It is realized by transparent material and preferably by polyethylene terephthalate (PET), more specifically by polyethylene terephthalate known with the initials PETG, or polycarbonate and is produced by injection moulding.

The window 2 has a substantially cylindrical shape having the distal face realizing the surface through which the lens 13 views the inside of the human body.

It has a diameter corresponding to the diameter of the tubular sheath 4, a height preferably between 2 mm and 10 mm and a wall thickness preferably between 0.3 mm and 0.5 mm.

The connection element 3 constrains the protective device 1 to the endoscopic apparatus 10.

It can preferably be constrained to the proximal portion of the probe 11, and in particular to the coupling element 14 of this probe 11, so that the protective device 1 can cover the entire probe 11.

For this purpose, the coupling element 3 has a cavity substantially countershaped to the coupling element 14 and comprises inner coupling grooves **3a** suitable to constrain this connection element 3 to the coupling element 14.

It is realized exteriorly by a cylindrical or truncated cone shaped element and has a coupling portion 3b suitable to allow gluing of the first portion 4a of the tubular sheath 4.

Operation of the protection device 1, the structure of which is described above, is as follows.

Before using the endoscopic apparatus 10, it is covered by means of the protective device 1.

The protective device 1 is suitably sterilized in advance and disposed in a sterile container and closed.

The device 1 is then inserted over the probe 11, so that the window 2 is superposed on the lens 13, the tubular sheath 4 is placed at the level of the central body of the probe 11 and the connection element 3 is fastened to the coupling element 14.

When the connection element 3 is fastened to the coupling element 14, the tubular sheath 4 is preferably elastically elongated in the axial direction, to allow the window 2 to remain in close contact with the lens 13 and thus to remain substantially in contact with the walls of the probe 11.

Finally, the protective device is discarded after use thereof.

To remove the device 1 from the apparatus 10 the connection element 3 must be released from the coupling element 14. This operation can be performed by hand by pulling the connection element 3 in the distal direction.

Subsequently, the device 1 is removed from the apparatus 10 by continuing to pull the connection element 3 in the distal direction.

Due to the described rigidity of the first portion 4a, during removal the connection element 3 draws with it the entire device 1.

Substantially, the drawback realized by the fact that the sheath 4 adheres closely to the probe 11 during removal of this sheath 4 does not occur.

The first portion 4a in fact is sufficiently rigid that it does not twist around itself during this operation and, moreover, draws the second portion 4b along the surface of the probe 11 during the removal operation.

The invention comprises a new process for producing a protective device 1 for endoscopic apparatus 10 as described above.

It comprises a step of immersing the first portion 4a in a deplasticizing liquid. Said deplasticizing liquid is preferably realized by an alcohol, and in particular an isopropyl alcohol, also called isopropanol or 2-propanol.

Said immersion step has a duration of between 2 and 15 minutes and in particular between 5 and 7 minutes.

The immersion step is subsequent to the step to produce the individual elements of which the device 1 is composed and prior to assembly thereof.

The invention achieves important advantages.

In fact, the device 1 allows simple removal thereof after use, without the need to add lubricating elements such as talc and the like.

Consequently, the sheath 4 can be maintained in close contact with the probe 11 and the window 2 in close contact with the lens 13.

Moreover, the device 1 does not therefore obstruct the apparatus 10 and in particular the probe 11.

In fact, the portion of the probe 11 which is inserted inside the human body and has to conform to the shape of this body, is realized by the distal portion of this probe 11, covered by the second portion 4b of the sheath 4.

Being very flexible, this second portion 4b is capable of easily following the deformations of the probe 11 and does not pose an obstacle for it.

A further advantage is realized by the fact that the device 1 is inexpensive, in particular if produced with the procedure described, which does not require particularly arduous or complex steps or gluing processes of the first and second portion 4a and 4b.

The invention is susceptible to modifications and variants falling within the inventive concept. All the details can be replaced by equivalent elements and the materials, the shapes and dimensions can be any.

## Claims

1. Protective device (1) for endoscopic apparatus (10), including an elongated and flexible probe (11) and comprising a lens (13) at the distal end, said device (1) comprising: a window (2), suitable to cover said lens (13), a connection element (3), suitable to constrain said protective device (1) to said endoscopic apparatus (10), a flexible tubular sheath (4) suitable to cover at least part of said probe (11), and connected at the ends to said window (2) and to said connection element (3) **characterized in that** said tubular sheath (4) comprises a first portion (4a), extending from said connection element (3) for a length of between 3 cm and 20 cm and a second portion (4b) complementary with respect to said first portion (4a), and **in that** said first portion (4a) has a greater flexural rigidity with respect to said second portion.

2. Device according to claim 1, wherein said first portion (4a) has a length of between 5 cm and 25 cm.

3. Device according to claim 1, wherein said first portion (4a) extends for a fraction of between one half and one tenth of said tubular sheath (4).

4. Device according to claim 1, wherein said first portion (4a) extends for a fraction of approximately one third of said tubular sheath (4).

5. Process for the realization of a protective device (1) for endoscopic apparatus (10), including an elongated and flexible probe (11) and comprising at one end a lens (13), said protective device (1) comprising: a window (2), suitable to cover said lens (13), a connection element (3), suitable to constrain said protective device (1) to said endoscopic apparatus (10), a tubular sheath (4) made of flexible polymer material suitable to cover said probe (11), and connected at the ends to said window (2) and to said connection element (3) and **characterized in that** it comprises a step of immersing a first portion (4a) of said tubular sheath, extending from said connection element (3) for a length of between 3 cm and 20 cm, in a deplasticizing liquid.

6. Process according to claim 5, wherein said immersion step has a duration of between 2 and 15 minutes.

7. Process according to claim 6, wherein said immersion step has a duration of between 5 and 7 minutes.

8. Process according to one or more of the previous claims, wherein said deplasticizing liquid is an alcohol.

9. Process according to claim 5, wherein said deplasticizing liquid is an isopropyl alcohol.
